# EUROPÄISCHE PATENTSCHRIFT

(11) **EP 1 363 658 B1**
(45) Veröffentlichungstag und Bekanntmachung des Hinweises auf die Patenterteilung: **17.11.2004**
(21) Anmeldenummer: 02714031.8
(22) Anmeldetag: 19.02.2002
(51) Int. Cl.: A61K 38/33

(54) **BETA-ENDORPHIN / CRF - GENTHERAPIE ZUR LOKALEN SCHMERZBEKÄMPFUNG**
BETA-ENDORPHIN / CRF - GENE THERAPY FOR COMBATTING PAIN LOCALLY
THERAPIE GENIQUE UTILISANT BETA-ENDORPHIN / CRF POUR COMBATTRE LA DOULEUR LOCALEMENT

(30) Priorität: 24.02.2001 DE 10109092
(43) Veröffentlichungstag der Anmeldung: 26.11.2003
(73) Patentinhaber: Mologen AG, 14195 Berlin (DE)
(72) Erfinder: WITTIG, Burghardt, 14129 Berlin (DE); STEIN, Christoph, 10825 Berlin (DE); SCHÄFER, Michael, 12205 Berlin (DE); SCHROFF, Matthias, 14057 Berlin (DE); JUNGHANS, Claas, 10551 Berlin (DE); KÖNIG MEREDIZ, Sven Andres, San Sebastian de los Reyes, Madrid (ES)
(74) Vertreter: Bergmann, Simon
(86) Internationale Anmeldenummer: PCT/DE2002/000583
(87) Internationale Veröffentlichungsnummer: WO 2002/067996

(56) Entgegenhaltungen:
- EP-A- 0 967 274
- WO-A-00/16800
- BAUER ET AL.: "EXPRESSION OF BIOLOGICALLY ACTIVE BETA-ENDORPHIN IN K562 CELLS" ABSTRACT SUN27, 31TH CONFERENCE 2000 INRC, 16. - 18. Juli 2000, XP002211427 Seattle, Washington, USA in der Anmeldung erwähnt
- TANELIAN D ET AL: "GENE THERAPY WITH ADENOVIRAL B-ENDORPHIN IS ANTINOCICEPTIVE" ANESTHESIOLOGY, AMERICAN SOCIETY OF ANESTHESIOLOGISTS, PHILADELPHIA, PA,, US, Bd. 85, Nr. 3A, September 1996 (1996-09), Seite A654 XP000867358 ISSN: 0003-3022
- SCHAFER M ET AL: "Expression of corticotropin-releasing factor in inflamed tissue is required for intrinsic peripheral opioid analgesia." PROCEEDINGS OF THE NATIONAL ACADEMY OF SCIENCES OF THE UNITED STATES OF AMERICA. UNITED STATES 11 JUN 1996, Bd. 93, Nr. 12, 11. Juni 1996 (1996-06-11), Seiten 6096-6100, XP002211428 ISSN: 0027-8424 in der Anmeldung erwähnt
- SCHÄFER M ET AL: "Interleukin 1 beta and corticotropin-releasing factor inhibit pain by releasing opioids from immune cells in inflamed tissue." PROCEEDINGS OF THE NATIONAL ACADEMY OF SCIENCES OF THE UNITED STATES OF AMERICA. UNITED STATES 10 MAY 1994, Bd. 91, Nr. 10, 10. Mai 1994 (1994-05-10), Seiten 4219-4223, XP002211429 ISSN: 0027-8424 in der Anmeldung erwähnt
- DATABASE EMBL [Online] EMBL; 28. Januar 1986 (1986-01-28) "EMBL:RNCRFR - Rat mRNA for corticotropin-releasing factor precursor (prepro-CRF)" retrieved from WWW.EBI.AC.UK Database accession no. X03036 XP002211430
- DATABASE EMBL [Online] EMBL; 29. Juli 1991 (1991-07-29) "EMBL:HSPOMC9 - Human proopiomelanocortin (POMC) gene, exon 3." retrieved from WWW.EBI.AC.UK Database accession no. J00292 XP002211431
- CHANG A C ET AL: "Structural organization of human genomic DNA encoding the pro-opiomelanocortin peptide." PROCEEDINGS OF THE NATIONAL ACADEMY OF SCIENCES OF THE UNITED STATES OF AMERICA. UNITED STATES AUG 1980, Bd. 77, Nr. 8, August 1980 (1980-08), Seiten 4890-4894, XP001095590 ISSN: 0027-8424
- DATABASE EMBL [Online] EMBL; 13. Juni 1985 (1985-06-13) "EMBL:RNPOMC3 - Rat proopiomelanocortin (POMC) gene, exon 3." retrieved from WWW.EBI.AC.UK Database accession no. J00759 XP002211432

### SEQUENCE LISTING

<110> Mologen Forschungs-, Entwicklungs- und Vertriebs GmbH
<120> Mittel zur lokalen Schmerzbehandlung
<130> XI 152/02
<150> DE 101 09 092.7
   <151> 2001-02-24
<160> 10
<170> PatentIn version 3.1
<210> 1
   <211> 535
   <212> DNA
   <213> synthetic sequence
<220>
   <221> Intron
   <222> (446)..(532)
   <223> β-endorphin cDNA sequence
<220>
   <221> Intron
   <222> (446)..(532)
   <223> β-endorphin cDNA sequence
<400> 1
   atgaacttgg agacaggcag ccggggctca gagttcggca tgagcgcagt gagctgcggc     60
   aatgggaaac atgccgagat tctgctacag tcgctcaggg gccctgctgc tggccctcct    120
   gcttcagacc tccatagacg tgtggagctg gtgcctggag agcagccagt gccaggacct    180
   caccacggaa agcaacctgc tggcttgcat ccgggcctgc agactcgacc tctcggcgga    240
   gacgcccgtg tttccaggca acggagatga acagcccttg actgaaaatc cccggaagta    300
   cgtcatgggt cacttccgct gggaccgctt cggcccgaga aacagcagca gtgctggcgg    360
   ctcagcgcag aggcgtgcgg aggaagagac ggcgggggga gatggccgtc cggagccaag    420
   tccacgggag ggcaagcgct acggcggctt catgacctcc gagaagagcc agacgcccct    480
   ggtgacgctc ttcaagaacg ccatcatcaa gaacgtgcac aagaagggcc agtga         535
<210> 2
   <211> 663
   <212> DNA
   <213> synthetic sequence
<220>
   <221> Intron
   <222> (376)..(462)
   <223> β-endorphin cDNA sequence
<220>
   <221> Intron
   <222> (475)..(562)
   <223> second β-endorphin CDNA sequence
<220>
   <221> Intron
   <222> (574)..(660)
   <223> third β-endorphin cDNA sequence
<400> 2
   atgccgagat tctgctacag tcgctcaggg gccctgctgc tggccctcct gcttcagacc     60
   tccatagacg tgtggagctg gtgcctggag agcagccagt gccaggacct caccacggaa    120
   agcaacctgc tggcttgcat ccgggcctgc agactcgacc tctcggcgga gacgcccgtg    180
   tttccaggca acggagatga acagcccttg actgaaaatc cccggaagta cgtcatgggt    240
   cacttccgct gggaccgctt cggcccgaga aacagcagca gtgctggcgg ctcagcgcag    300
   aggcgtgcgg aggaagagac ggcgggggga gatggccgtc cggagccaag tccacgggag    360
   ggcaagcgct acggcggctt catgacctcc gagaagagcc agacgcccct ggtgacgctc    420
   ttcaagaacg ccatcatcaa gaacgtgcac aagaagggcc agaagcgcta cggcggcttc    480
   atgacctccg agaagagcca gacgcccctg gtgacgctct tcaagaacgc catcatcaag 540
   aacgtgcaca agaagggcca gaagcgctac ggcggcttca tgacctccga gaagagccag 600
   acgcccctgg tgacgctctt caagaacgcc atcatcaaga acgtgcacaa gaagggccag 660
   tga 663
<210> 3
   <211> 708
   <212> DNA
   <213> Rat
<400> 3
   atgccgagat tctgctacag tcgctcaggg gccctgctgc tggccctcct gcttcagacc 60
   tccatagacg tgtggagctg gtgcctggag agcagccagt gccaggacct caccacggaa 120
   agcaacctgc tggcttgcat ccgggcctgc agactcgacc tctcggcgga gacgcccgtg 180
   tttccaggca acggagatga acagcccttg actgaaaatc cccggaagta cgtcatgggt 240
   cacttccgct gggaccgctt cggcccgaga aacagcagca gtgctggcgg ctcagcgcag 300
   aggcgtgcgg aggaagagac ggcgggggga gatggccgtc cggagccaag tccacgggag 360
   ggcaagcgct cctactccat ggagcacttc cgctggggca agccggtggg caagaagcgg 420
   cgccctgtga aggtgtaccc caatgtcgcc gagaacgagt cggccgaggc ctttccccta 480
   gagttcaaga gggagctgga aggcgagcag cctgatggct tggagcacgt cctggagccg 540
   gataccgaga aggccgacgg gccctatcgg gtggagcact tccgctgggg caacccgccc 600
   aaggacaagc gctacggcgg cttcatgacc tccgagaaga gccagacgcc cctggtgacg 660
   ctcttcaaga acgccatcat caagaacgtg cacaagaagg gccagtga 708
<210> 4
   <211> 615
   <212> DNA
   <213> synthetic sequence
<400> 4
   atgccgagat tctgctacag tcgctcaggg gccctgctgc tggccctcct gcttcagacc 60
   tccatagacg tgtggagctg gtgcctggag agcagccagt gccaggacct caccacggaa 120
   agcaacctgc tggcttgcat ccgggcctgc agactcgacc tctcggcgga gacgcccgtg 180
   tttccaggca acggagatga acagcccttg actgaaaatc cccggaagta cgtcatgggt 240
   cacttccgct gggaccgctt cggcccgaga aacagcagca gtgctggcgg ctcagcgcag 300
   aggcgtgcgg aggaagagac ggcgggggga gatggccgtc cggagccaag tccacgggag 360
   ggcaagcgct cctactccat ggagcacttc cgctggggca agccggtggg caagaagcgg 420
   cgccctgtga aggtgtaccc caatgtcgcc gagaacgagt cggccgaggc ctttccccta 480
   gagttcaaga gggagctgga aggcgagcag cctgatggct tggagcacgt cctggagccg 540
   gataccgaga aggccgacgg gccctatcgg gtggagcact tccgctgggg caacccgccc 600
   aaggacaagc gctga 615
<210> 5
   <211> 12
   <212> PRT
   <213> Simian virus 40
<400> 5
   Pro Lys Lys Lys Arg Lys Val Glu Asp Pro Tyr Cys
   1 5 10
<210> 6
   <211> 1195
   <212> DNA
   <213> Rat
<400> 6
   aaactcagag cccaagtacg ttgagaaact gaagagaaag gggaaaggca aagaaaagga 60
   gaagagaaag gagaagagga agaaaacctg caggaggcat cctgagagag gtacctcgca 120
   gaacaacagt gcgggctcac ctgccaaggg aggagaagag agcgccccta aacatgcggc 180
   tgcggctgct ggtgtccgcg ggcatgctgc tggtggctct gtcgccctgt ctgccttgca 240
   gggccctgct gagcagggga tccgtctctg gagcgccgcg ggccccgcag ccgttgaatt 300
   tcttgcaacc ggagcagccc cagcaacctc agccgattct gatccgcatg ggtgaagaat 360
   acttcctccg cctggggaac ctcaacagaa gtcccgctgc tcggctgtcc cccaactcca 420
   cgcccctcac cgcgggtcgc ggcagccgcc cctcgcacga ccaggctgcg gctaactttt 480
   tccgcgtgtt gctgcagcag ctgcagatgc ctcagcgccc gctcgacagc agcacggagc 540
   tggcggaacg cggcgccgag gatgccctcg gtggccacca gggggcgctg gagagggaga 600
   ggcggtccga ggagccgccc atctctctgg atctcacctt ccaccttctg agggaagtct 660
   tggaaatggc cagggcagag cagttagctc agcaagctca cagcaacagg aaactgatgg 720
   agattatcgg gaaatgaaat gttgcgcttg gccaaaacga ttctgcattt agcacacaag 780
   taaaaataaa aaatttaaaa cacagtattc tgtaccatac tgcagctctg atatcatttg 840
   tttattttta tatagcttga agcatagaag atgtacaggg agagagccta tatacccctt 900
   aattagcatg cacaaagtgt gtttctttgt agtaacaaaa cagcgttatt tgtattgttc 960
   acgcttagtt tctatgtgca aataagtgtc tttatagcga tatcttaaag aaaatgtgga 1020
   tccaaggagg aaacctttaa aaaagcagat ggaagtcacc cagttgtttt tatttggaga 1080
   cacagtgtaa gagaattcat tcttgagggg tggctaggac aaaatgtgta agctctttga 1140
   atcaactttt tcttgtaaat gtttcaataa taaaacatct ttctgatcct tggtc 1195
<210> 7
   <211> 564
   <212> DNA
   <213> synthetic sequence
<220>
   <221> Intron
   <222> (376)..(462)
   <223> β-endorphin cDNA sequence
<220>
   <221> Intron
   <222> (475)..(562)
   <223> second β-endorphin cDNA sequence
<400> 7
   atgccgagat tctgctacag tcgctcaggg gccctgctgc tggccctcct gcttcagacc 60
   tccatagacg tgtggagctg gtgcctggag agcagccagt gccaggacct caccacggaa 120
   agcaacctgc tggcttgcat ccgggcctgc agactcgacc tctcggcgga gacgcccgtg 180
   tttccaggca acggagatga acagcccttg actgaaaatc cccggaagta cgtcatgggt 240
   cacttccgct gggaccgctt cggcccgaga aacagcagca gtgctggcgg ctcagcgcag 300
   aggcgtgcgg aggaagagac ggcgggggga gatggccgtc cggagccaag tccacgggag 360
   ggcaagcgct acggcggctt catgacctcc gagaagagcc agacgcccct ggtgacgctc 420
   ttcaagaacg ccatcatcaa gaacgtgcac aagaagggcc agaagcgcta cggcggcttc 480
   atgacctccg agaagagcca gacgcccctg gtgacgctct tcaagaacgc catcatcaag 540
   aacgtgcaca agaagggcca gtga 564
<210> 8
   <211> 936
   <212> DNA
   <213> Homo sapiens
<220>
   <221> Intron
   <222> (847)..(933)
   <223> β-endorphin cDNA sequence
<400> 8
   atgccgagat cgtgctgcag ccgctcgggg gccctgttgc tggccttgct gcttcaggcc    60
   tccatggaag tgcgtggctg gtgcctggag agcagccagt gtcaggacct caccacggaa   120
   agcaacctgc tgaaggggat gggacaaaag aggcggtggc aagatcttag atgcccacga   180
   gtgccaagaa agcaggtggg cagacctgcc tgtagggagg cctcgacgct tgacacgccc   240
   gacactgtgc cctgtgtcct cggcgagtgc atccgggcct gcaagcccga cctctcggcc   300
   gagactccca tgttcccggg aaatggcgac gagcagcctc tgaccgagaa cccccggaag   360
   tacgtcatgg gccacttccg ctgggaccga ttcggccgcc gcaacagcag cagcagcggc   420
   agcagcggcg cagggcagaa gcgcgaggac gtctcagcgg gcgaagactg cggcccgctg   480
   cctgagggcg gccccgagcc ccgcagcgat ggtgccaagc cgggcccgcg cgagggcaag   540
   cgctcctact ccatggagca cttccgctgg ggcaagccgg tgggcaagaa gcggcgccca   600
   gtgaaggtgt accctaacgg cgccgaggac gagtcggccg aggccttccc cctggagttc   660
   aagagggagc tgactggcca gcgactccgg gagggagatg gccccgacgg ccctgccgat   720
   gacggcgcag gggcccaggc cgacctggag cacagcctgc tggtggcggc cgagaagaag   780
   gacgagggcc cctacaggat ggagcacttc cgctggggca gcccgcccaa ggacaagcgc   840
   tacggcggtt tcatgacctc cgagaagagc cagacgcccc tggtgacgct gttcaaaaac   900
   gccatcatca agaacgccta caagaagggc gagtga                             936
<210> 9
   <211> 87
   <212> DNA
   <213> RAT
<400> 9
   ggcttcatga cctccgagaa gagccagacg cccctggtga cgctcttcaa gaacgccatc    60
   atcaagaacg tgcacaagaa gggccag                                        87
<210> 10
   <211> 87
   <212> DNA
   <213> Human
<400> 10
   ggtttcatga cctccgagaa gagccagacg cccctggtga cgctgttcaa aaacgccatc    60
   atcaagaacg cctacaagaa gggcgag                                        87

## Beschreibung

Die Erfindung betrifft ein Mittel zur Dämpfung der Schmerzempfindung, insbesondere bei akuten und chronischen entzündlichen Erkrankungen, durch Expression körpereigener neuroendokriner Peptide an der Entzündungsstelle.

### Allgemeiner Hintergrund der Erfindung

Schmerz ist ein Signal des Körpers, das Krankheit oder Verletzung anzeigt. Als solches ist es, obwohl subjektiv als unangenehm empfunden, sinnvoll und wichtig. Wenn Schmerz chronisch wird, tritt seine Wamfunktion in den Hintergrund. Chronischer Schmerz ist ein medizinisch und sozio-ökonomisch schwerwiegendes Problem. In Deutschland allein leben 700.000 bis 800.000 Schmerzkranke, von denen nur 10% adäquat behandelt werden können. Zu den wichtigsten Verursachern von Schmerzerkrankungen gehören die Gruppe der rheumatischen Erkrankungen, Krebsleiden sowie Schmerzen unklarer Ursache wie Rücken- und Kopfschmerzleiden ohne feststellbare organische Ursache. Allein rheumatische Schmerzleiden verursachen in Deutschland jährlich Kosten von rund 30 Milliarden Mark. In den USA leiden zur Zeit ca. 40 Millionen Menschen an Arthritis, für 2020 ist eine Fallzahl von fast 60 Millionen geschätzt worden (J Managed Care Pharm 1999: 414-419).

Neben die Behandlung der den chronischen Schmerz verursachenden grundlegenden Leiden (welche in vielen Fällen an einem mangelnden Verständnis der pathogenen Mechanismen oder einem Fehlen an therapeutischen Interventionsmöglichkeiten scheitert) tritt die Notwendigkeit, den Schmerz wirkungsvoll zu bekämpfen. Die beiden Substanzklassen, die hier vor allem eingesetzt werden, sind die der Opiate und der sog. nicht-steroidalen Entzündungshemmer (Nonsteroidal antiinflammatoy drugs, NSAIDs).

Beide Substanzklassen sind in ihrer Anwendung nicht unproblematisch, wegen des tatsächlichen oder subjektiv befürchteten Suchtpotentials, der möglichen Atemdepression, Übelkeit und Sedierung (Opiate) oder der z.T. dramatischen Nebenwirkungen vor allem im gastrointestinalen Bereich (Ulcus, Blutungen (NSAIDs)). Zusätzlich zu den durch den chronischen Schmerz verursachten Beschwerden besteht für die mit NSAIDs behandelten Patienten eine erheblich erhöhte Wahrscheinlichkeit, an gastrointestinalen Beschwerden als Folge der Therapie zu leiden.

Das Bedürfnis nach lokal (am Ort der Schmerzentstehung) wirksamen Mitteln ohne die genannten zentralnervösen oder gastrointestinalen Nebenwirkungen zur Schmerzbekämpfung ist mithin offensichtlich.

### Wissenschaftliche Vorarbeiten

Seit ca. 1990 ist bekannt, dass Opioidrezeptoren in der Peripherie zur Schmerzdämpfung (Antinociception) in entzündetem Gewebe beitragen, und dass die wirksame Komponente der Antinociception körpereigenes β-Endorphin (β-END) ist (Stein et al., Proc. Nat. Acad. Sci. USA 87, 5935-5939 (1990)). Der Effekt ist im Modell der entzündeten Rattenpfote auf entzündetes Gewebe beschränkt (Stein et al. J. Neuroscience 10, 1292-1298 (1990)). Das β-END wird im entzündeten Gewebe von Lymphozyten produziert und freigesetzt (Cabot et al. J. Clin. Invest. 100, 142-148 (1997)).

Die lokale Freisetzung von Corticotropin-Releasing Factor (CRF) ist dabei Voraussetzung des schmerzdämpfenden Effektes des β-END, und für Interleukin-1β konnte eine den antinociceptiven Effekt fördernde Funktion gezeigt werden (Schäfer et al. Proc. Nat. Acad. Sci. USA 91, 4219-4223; ibid. 93, 6096-6100 (1996)). Die antinociceptive Wirkung der Leukozyten bedarf der Einwanderung der β-END sezernierenden Zellen in das entzündete Gewebe (Machelska et al, Nature Ishii et al. (Experimental Neurology 166, 90-98 (2000)) konnten zeigen, dass die Implantation von β-END produzierenden Tumorzellinien in den Subarachoindalraum zur Schmerzdämpfung im Tiermodell führt. Ähnliche Ergebnisse zeigen Wu et al. (Neural Transplant Plast 4, 15-26 (1993)). Es ist dabei zu bemerken, dass diese Untersuchungen nicht an Tieren mit akut entzündetem Gewebe durchgeführt wurden, und die Implantation von genetisch modifizierten Tumorzellen ins zentrale Nervensystem eine relativ grosse Ferne von in Menschen anwendbaren Problemlösungen erkennen lässt.

Feingold und ladarola (Hum Gene Therapy 10(7):1251-7 (1999)) zeigten die antinociceptive Wirkung von β-END, welches von genetisch modifizierten Adenoviren sezerniert wird, die in den Subarachnoidalraum gespritzt wurden und die Meningen infizierten. Diese Methodik wurde von ladarola et al. ebenfalls zum Patent angemeldet (WO 0016800 A2). Eine virale Methode des Gentransfers beschreiben Wilson et al. (Proc Natl Acad Sci U S A 96, 3211-6 (1999); Brain Res 792, 133-5 (1998)), die Herpesviren als Genfähre in Gewebe des Zentralen Nervenssystems einsetzen.

β-END ist ein 31 Aminosäuren langes Peptid. Es ist kodiert auf dem Exon 3 des Pro-Opiomelanocortingens (POMC) und wird als Teil eines Vorläuferpeptides gebildet, aus dem auch die Peptidhormone ACTH und gamma-Lipotropin erhalten werden. Die Transkriptlänge des POMC ist unterschiedlich für zentrale und periphäre Gewebe (Grauerholz et al., Peptides 19, 939-948 (1998)).

Es wurde bereits gezeigt (Bauer M. et al., Abstract Sun27, 31th Conference 2000 INRC, July 16^{th} to July 18^{th} 2000, Seattle, Washington USA), Zellkulturzellen mit β-Endorphin-(β-END) exprimierenden Konstrukten zu transfizieren und anschließend das Zelllysat dieser transfizierten Zellen in Rattenpfoten zu injizieren. Es wurde also rekombinantes END, verunreinigt mit zellulären Bestandteilen wie Proteinen, Membranfragmenten, Organellen und Nukleinsäuren, in Rattenpfoten injiziert. Dieser Versuch diente dazu, um ganz grundsätzlich festzustellen, ob von den Konstrukten überhaupt END exprimiert wird, wenn es in analoger Verfahrensweise zur biotechnologischen Herstellung rekombinanter Proteine, aber ohne die pharmazeutisch dazugehörende umfangreiche Aufreinigung, in Zellkulturzellen gebildet wird. Es konnte nur in den Zelllysaten β-Endorphin nachgewiesen werden, die mit einem Vektor transfizierte wurden, der die vollständige POMC-cDNA-Sequenz der Ratte als kodierenden Bereich enthielt. Die Injektion dieser Lysate war geeignet, um in einem "Rattenpfoten-Drucktest" einen schmerzlindernden Effekt nachweisen zu können. Dieser Versuch war jedoch nicht dazu geeignet, um festzustellen, ob die vom pharmazeutisch-anwendungstechnicshen Standpunkt einzig tolerable Verwendung von isolierten Expressionskonstrukten zur Inkektion in entzündetes Gewebe einen schmerzlindernden Effekt hat.

Ausgehend von diesem Stand der Wissenschaft ist es Aufgabe der vorliegenden Erfindung, ein effektives Mittel zur Verminderung bzw. Unterdrückung der Schmerzempfindung bei Säugern, insbesondere Menschen, zur Verfügung zu stellen.

Gelöst wird diese Aufgabe durch die kennzeichnenden Merkmale des Anspruchs 1.

Erfindungsgemäß wird diese Aufgabe durch ein Mittel gelöst, welches - unter Ausschluß von Zellen oder Zellysaten - Expressionskonstrukte zur lokalen Expression von neuroendokrinen Peptiden oder funktionellen Teilen derselben beinhaltet. Insbesondere der Verzicht auf Expressionskonstrukte, die zum Zwecke der späteren Vakzinierung / Injizierung in Zellen verbracht wurden oder in Zellysaten enthalten sind, sondern in isolierter Form als "nackte DNA" oder komplexiert mit Polymeren injiziert werden, führt zu überraschenden Erfolgen.

Ein wesentlicher Aspekt der Erfindung besteht in der Unterdrückung von Schmerz durch die lokale Expression von neuroendokrinen Peptiden. Ein weiterer wesentlicher Aspekt der Erfindung besteht darin, geeignete Expressionskonstrukte zur lokalen Synthese von neuroendokrinen Peptiden, insbesondere β-Endorphin durch geeignete Abwandlungen von dessen Vorläufer POMC sowie Corticotropin-releasing-factor zur Verfügung zu stellen.

Erfindungsgemäß ist daher ein Mittel zur Verminderung oder Unterdrückung der Schmerzempfindung vorgesehen, welches Expressionskonstrukte zur Expression von neuroendokrinen Peptiden oder funktionellen Teilen derselben enthält, wobei es sich insbesondere um β-Endorphin aus Abwandlungen des Vorläufers Pro-Opiomelanocortingens (POMC) und/oder den Corticotropin-releasing-factor (CRF) handelt.

Das Mittel enthält erfindungsgemäß ein Genkonstrukt, welches zumindest den immediate-early Promotor des Cytomegalievirus (CMV-Promoter), die DNA-Sequenz des Pro-Opiomelanocortingens (POMC), aus der die kodierenden Abschnitte für die Gewebshormone adrenocorticotropes Hormon (ACTH) und/oder melanozytenstimulierendes Hormon (beta-MSH) deletiert wurden, und das zumindest einen für β-Endorphin kodierenden Sequenzabschnitt enthält sowie eine geeignete Polyadenylierungssequenz enthält.

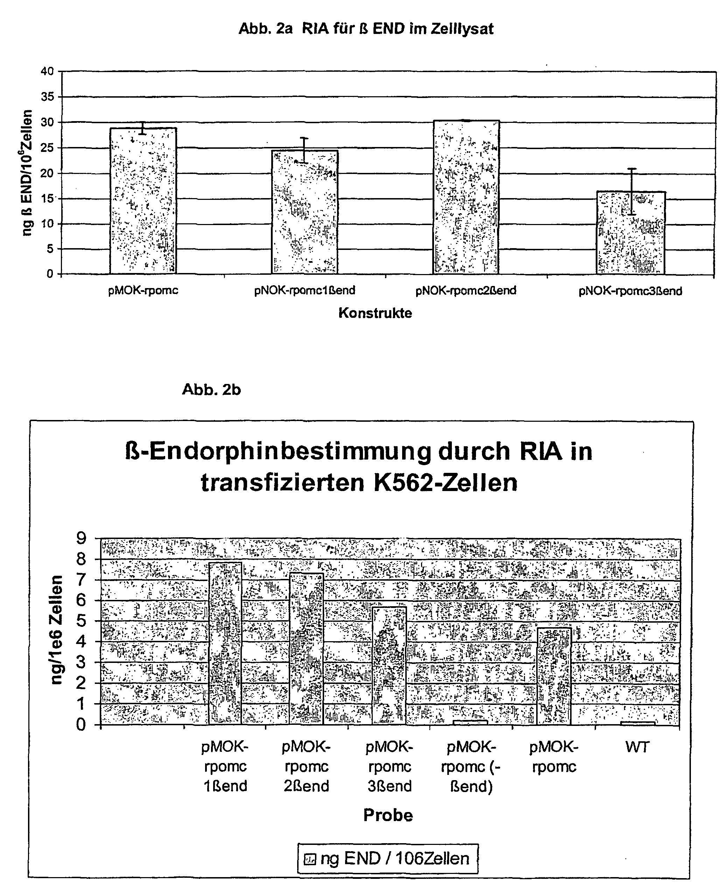

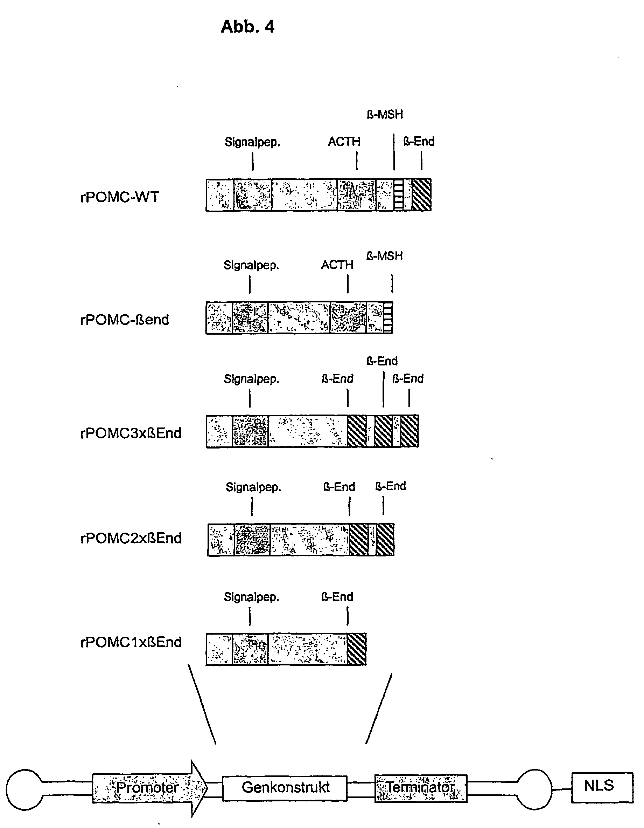

Da die Expression der Wildtyp POMC-Sequenz in Gewebe neben der für die Schmerzdämpfung erwünschten Synthese von β-END auch zur Synthese der Gewebshormone adrenocoticotropes Hormon (ACTH) und beta-melanozytenstimulierendes Hormon (beta-MSH) führt und nicht ausgeschlossen werden kann, dass in der weiteren klinischen Entwicklung der hier beschriebenen Erfindung sich die Synthese dieser "Nebenprodukte" als nachteilhaft erweist, wurden Expressionskassetten hergestellt, in denen die für ACTH und beta-MSH kodierenden Abschnitte deletiert oder durch β-END kodierende Abschnitte ersetzt wurden (Beispiele 1.3, 1.4 und Abb. 4). Auch diese Expressionskassetten zeigen im RIA die Synthese von β-END. (Abb. 2a und 2 b).

Insbesondere diejenigen Expressionskassetten, in denen die für ACTH und beta-MSH kodierenden Abschnitte durch β-END kodierende Abschnitte (2xβ-END bzw. 3xβ-END) ersetzt wurden, stellen eine bevorzugte Ausführungsform der Erfindung dar, da dieses Expressionsergebnis nicht zu erwarten und daher überraschend ist. Aus der natürlichen POMC-mRNA-Sequenz wird ein Polypeptid translatiert, welches von endogenen Proteasen in die wirksamen Peptidhormone gespalten wird. Die Proteasen erkennen dabei "chemisch" den Sequenzkontext der an der Spaltstelle gelegenen Aminosäuren. Es ist insofern sehr überraschend, dass der Austausch der natürlichen Bestandteile des POMC-Gens, welche kein Endorphin kodieren, durch weitere Endorphin-kodierende Bestandteile, zu einer erhöhten Produktion von Endorphin durch diese Konstrukte exprimierende Zellen führt. Dies ist insbesondere deshalb überraschend, weil nicht zu erwarten war, dass die im Vergleich zur Originalsequenz geänderte Aminosäurensequenz an den gleichen Stellen zu korrekter Proteaseaktivität führt.

Die erfinderische Leistung liegt daher - unter anderem - darin, dass eine "Austauschklonierung" vorgenommen wurde, indem die ohnehin in der Sequenz des natürlichen Leserahmens der POMC-Sequenz mehrfach hintereinander angeordneten Sequenzabschnitte (β-END - ACTH - beta-MSH) durch mehrfache β-END Sequenzabschnitte ersetzt bzw. ausgetauscht wurden (ACTH > β-END und beta-MSH > β-END), so dass im Ergebnis innerhalb der natürlichen POMC-Sequenz dann die Sequenzabschnitte für β-END - β-END - β-END einander folgen und auch exprimiert werden. Die Erfindung macht sich daher die natürliche Sequenz zunutze.

**Tabelle 1:**

| Übersicht der in dieser Anmeldung aufgeführten Sequenzen. Die Nummerierung der Sequenzen entspricht der des Sequenzprotokolls: | |
|---|---|
| Sequenznummer | Bezeichnung |
| Seq. ID 1 | rPOMC 1x β-Endorphin |
| Seq. ID 2 | rPOMC 3x β-Endorphin |
| Seq. ID 3 | POMC Ratte (rPOMC-WT) |
| Seq. ID 4 | rPOMC ohne β-Endorphin |
| Seq. ID 5 | NLS aus SV-40 |
| Seq. ID 6 | CRF Ratte (rCRF) |
| Seq. ID 7 | rPOMC 2x β-Endorphin |
| Seq. ID 8 | POMC human |
| Seq. ID 9 | β-Endorphin Sequenz Ratte |
| Seq. ID 10 | β-Endorphin Sequnez Mensch |

Erfindungsgemäß ist daher auch Gegenstand der Anmeldung eine Desoxyribonukleinsäuresequenz, enthaltend zwei der für β-Endorphin kodierenden Sequenzabschnitte des Pro-Opiomelanocortingens (POMC), nämlich die in Seq.ID 7 (rPOMC 2xβ-END) und im folgenden wiedergegebene Sequenz.

Erfindungsgemäß ist aber auch Gegenstand der Anmeldung eine Desoxyribonukleinsäuresequenz, enthaltend drei der für β-Endorphin kodierenden Sequenzabschnitte des Pro-Opiomelanocortingens (POMC), nämlich die in Seq.ID 2 (rPOMC 3xβ-END) und im folgenden wiedergegebene Sequenz.

Erfindungsgemäß ist ferner auch Gegenstand der Anmeldung eine humane Desoxyribonukleinsäuresequenz, enthaltend den für β-Endorphin kodierenden Sequenzabschnitt des Pro-Opiomelanocortingens (POMC), nämlich die in Seq.ID 8 (human POMC) und im folgenden wiedergegebene Sequenz.

Der für β-Endorphin kodierende Sequenzabschnitt des Pro-Opiomelanocortingens (POMC), weißt die folgende DNA-Sequenz auf (siehe Seq.ID 9):

Die humane Sequenz (siehe Seq.ID 10): unterscheidet sich in lediglich 6 Basen von der Rattensequenz (unterstrichen).

Ein entsprechendes Expressionskonstrukt für den Corticotropin-releasing-factor (CRF) ist ebenfalls Gegenstand der Erfindung.

β-Endorphin kann durch Insertion der vollständigen Sequenz des POMC-Gens der Ratte (Seq.ID 3) zwischen den immediate-early Promotor des Cytomegalievirus (CMV-Promoter) und eine geeignete Polyadenylierungssequenz innerhalb eines Expressionsplasmides (Abb. 1) und nachfolgende Transfektion dieses Expressionsplasmides in Zellkulturzellen der Ratte oder des Menschen im Radioimmunoassay (RIA) nachgewiesen werden (Abb. 2a und 2b). Eine zur Kontrolle hergestellte Expressionskassette, in der vor der das β-END kodierenden Nukleinsäuresequenz ein Stop-Kodon eingeführt wurde, zeigt keine Expression von β-END im RIA (Abb. 3, rechter Balken).

Ein weiterer Aspekt der Erfindung betrifft Vektoren zur Herstellung der Expressionskonstrukte. Dabei wurden die Plasmide pMOK und pNOK verwendet, welche die DNA-Sequenzen bzw. den Sequenzabschnitt für β-Endorphin (β-END) in unterschiedlichen Variationen, nämlich mehrfach, enthält, aber auch die Sequenz für den Corticotropin-releasing-factor (CRF) enthielten. Das rPOMC ist ein Polyprotein, aus dem durch Abspaltung durch zelleigene Proteinasen die eigentlichen Peptidhormone (β-MSH, ACTH, βEndorphin) herausgespalten werden. Durch Ersatz der Sequenzen von βMSH und ACTH durch β-Endorphin erhofft man sich eine höhere Expression des β-END und dadurch eine verstärkte Schmerzreduktion. Die mehrfache Nutzung des Sequenzabschnittes für β-Endorphin hat den Sinn, dass verstärkt β-Endorphin abgelesen und exprimiert wird (siehe oben).

Es wurden verschiedene Expressionskonstrukte aus dem Plasmid pMOK-POMC hergestellt. Die Einzelheiten der Herstellung sind in den der EP 0 941 318 B1 und DE 198 26 758 offenbart. Im Einzelnen wurden Plasmid-DNA, linear-kovalent geschlossene unmodifizierte (sogenannte "MIDGE"-) Expressionskonstrukte sowie mit einem die Kemlokalisationssequenz (NLS) des large T-Antigens von SV40 enthaltenden Peptid modifizierte MIDGE-Konstrukte hergestellt (MIDGE-NLS) (Abb. 4). Diese Modifikation zeigt den überraschende Vorteil, dass derart modifizierte Konstrukte die Transfektionseffizienz verstärken. Die NLS-Kopplung der minimalistischen Nukleinsäurekonstrukte führt zu einer deutlichen Erhöhung der Effizienz des Gen-Transfers und damit der Expression von β-END. Diese Expressionssteigerung wird dadurch erreicht, dass an das zu exprimierende Gen Oligodesoxyribonukleotide ligiert werden, an die zuvor ein nukleäres Lokalisationssignal (NLS) kovalent gebunden wurde. Wie aus *in vitro*-Versuchen bekannt ist, kann diese aus dem Virus SV40 stammende Sequenz den Transport von DNA aus dem Cytosol der Zelle in den Zellkern verbessern und somit die Transkriptionsrate der DNA steigern (vgl. WO 00/37659).

Es sind aber gleichsam auch grundsätzlich peptide bevorzugt, die mit den linear-kovalent geschlossenen Expressionskonstrukten konjugiert sind. Bevorzugt sind dabei kathionische Peptide mit einer aus 8 bis 20 Aminosäuren bestehenden Länge.

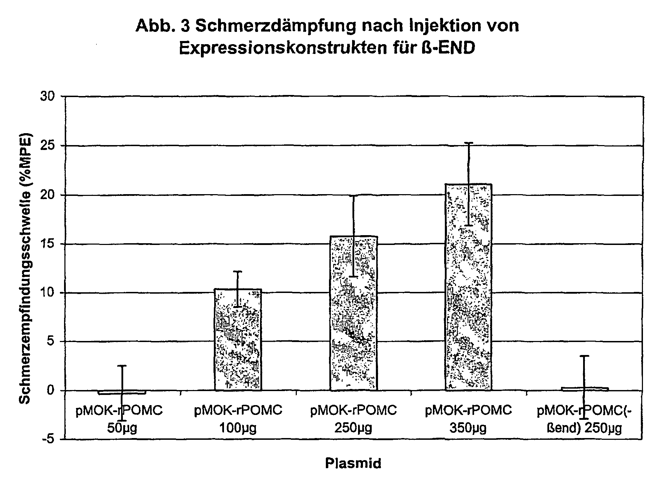

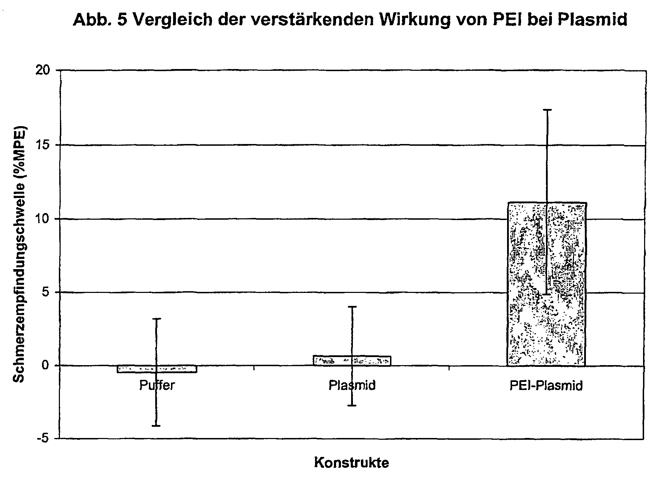

Bereits die Injektion von Plasmiden, welche die Expressionskassette für POMC enthalten, in entzündete Rattenpfoten, führt zu einer deutlichen Abnahme des Schmerzempfindens bei den behandelten Tieren (siehe Abb. 3). Diese Wirkung ist durch Komplexierung der DNA durch Polyethylenimin (PEI) noch deutlich verstärkbar (Abb. 5). Durch Komplexierung der DNA mit positiv geladenen Makromolekülen wird die Aufnahme von DNA in die Zelle erleichtert, zum anderen wird eine Art Kemtransportfunktion angenommen (Chemin I.et al.,J. Viral Hepat, Nov; 5 (6): 369-75, 1998).

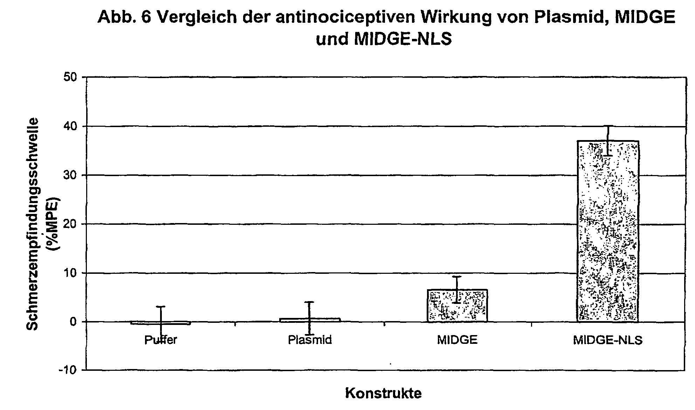

Die Injektion von POMC-kodierendem MIDGE führt ebenfalls zu einer signifikanten Reduktion des Schmerzempfindens. Die Injektion von peptidmodifizierten Konstrukten (MIDGE-NLS) allerdings hat mit grossem Abstand die höchste Wirkung (Abb.6).

Folgerichtig wird auch die Verwendung des erfindungsgemäßen Mittels, enthaltend die erfindungsgemäßen Expressionskonstrukte als Arzneimittel, insbesondere als Vakzine beansprucht.

Die Erfindung weißt insbesondere folgende Vorteile auf: Durch das erfindungsgemäße Mittel wird eine Behandlung von chronischen Schmerzleiden ermöglicht, bei der auf den Einsatz von Opiaten und der nicht-steroidalen Entzündungshemmenr (Nonsteroidal antiinflammatories, NSAIDs) verzichtet werden kann. Die mit derartigen Mitteln auftretenden bekannten Nebenwirkungen wie Übelkeit, Atemdepression, Abhängigkeit und Suchtprobleme (bei Opiaten), Magengeschwüre etc. können mithin vermieden werden. Ferner weißt das erfindungsgemäße Mittel den überraschende Vorteil auf, daß es länger anhaltend ist, nämlich im Bereich von Tagen als bei der Injektion von rekombinanten β-END (Wirkungsdauer im Bereich von Minuten).

Die vorliegende Erfindung schafft mithin ein Mittel zur Schmerzunterdrückung, wobei - im Gegensatz zu Bauer M. et al. (Abstract Sun27, 31th Conference 2000 INRC, July 16^{th} to July 18^{th} 2000, Seattle, Washington USA) - keine Transfizierung von DNA-Expressionskonstrukten in Zellkulturzellen und die nachfolgende Verwendung der rohen Proteinsuspension, sondern eine Injektion von exprimierbaren Konstrukten direkt in das entzündete Gewebe zur Schmerzunterdrückung stattfindet. Genau dieser Effekt konnte von Bauer et. al. nicht gezeigt werden. Es werden also keine transfizierten Zellen oder Zellysate als Bestandteil des erfindungsgemäßen Mittels bzw. der daraus resultierenden Vakzine verwendet. Die Vermeidung der Zellysate führt zu überraschenden Erfolgen, wie im folgenden näher noch ausgeführt wird. Überraschenderweise war der Effekt mit MIDGE noch besser als mit Plasmid, und noch viel besser mit den MIDGE-NLS-Konstrukten. Dieser Effekt insbesondere wird bei der Verwendung von Zellysaten nicht beobachtet.

Weitere vorteilhafte Maßnahmen sind in den übrigen Unteransprüchen beschrieben; die Erfindung wird anhand von Ausführungsbeispielen und den nachfolgenden Abbildungen näher beschrieben:

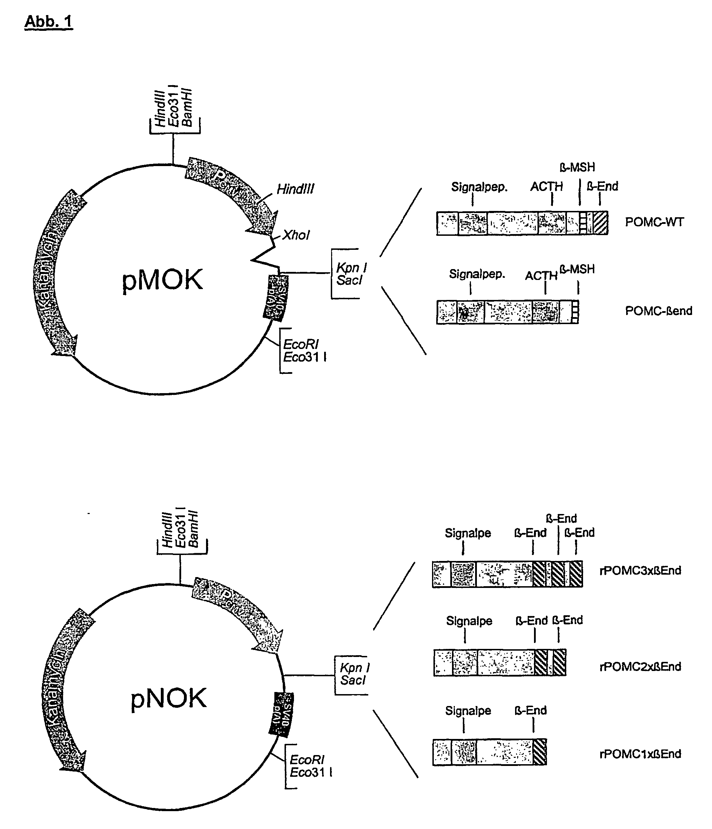

**Abb. 1** zeigt den funktionellen Aufbau der verwendeten Expressionsplasmide pMOK und pNOK sowie die klonierten rPOMC-Sequenzen. Die künstlichen Sequenzen rPOMC-1βEnd, rPOMC-2βEnd und rPOMC-3βEnd wurden in das Plasmid pNOK kloniert, das einen einfachen CMV Promotor und kein Intron besitzt.
**Abb. 2** zeigt den Nachweis der Expression von β-END im Zelllysat von Rattenzellkulturen mittels eines Radioimmunoassays (RIA). Alle Expressionskonstrukte zeigten eine deutliche Expression von βEND. Auch dieses Ergebnis der erhöhten Expression mit den künstlichen Konstrukten war nicht zu erwarten und steht in Gegensatz zu Bauer M. et al. (Abstract Sun27, 31th Conference 2000 INRC, July 16^{th} to July 18^{th} 2000, Seattle, Washington USA), wo lediglich das natürlich vorkommende POMC verwendet wurde. Dabei zeigen die Ergebnisse, dass Exprimierung nach Klonierung in den pMOK-Vektor (**Abb. 2b**) effektiver ist als wenn in den pNOK-Vektor kloniert wird (**Abb. 2a**).
**Abb. 3** zeigt die lineare Korrelation zwischen der applizierten Menge des Plasmids pMOK-rPMOC und der Schmerzempfindungsschwelle der Versuchstiere. Als Plasmidinsert wurde die vollständige rPOMC Sequenz (Bsp: 1.1) benutzt und das Plasmid in verschiedenen Konzentrationen in die Entzündungsstelle injiziert. Zum Nachweis der schmerzlindernden Wirkung von β- END, wurde eine um die Sequenz von β-END verkürzte Expressionskassette (Bsp. 1.2) hergestellt. Erwartungsgemäß zeigt sich keine schmerzlindernde Wirkung im Versuch, siehe rechten äußeren Balken in Abb. 3.
**Abb. 4** zeigt die verschiedenen Expressionskassetten, die zur Herstellung der MIDGE Konstrukte verwendet wurden. Die erzeugten Konstrukte enthalten neben der kodierenden Sequenz lediglich die zu ihrer Expression notwendigen Promotor- und Terminationssequenzen. Die Kopplung mit den Peptiden zur nukleären Lokalisation (NLS-Sequenz) erfolgte wahlweise.
**Abb. 5** zeigt ist die signifikante Verstärkung der Expression von β-END durch Komplexierung der DNA mit PEI bei Plasmid.
**Abb. 6** zeigt den Vergleich der antinociceptiven Wirkung der eingesetzten Expressionkonstrukte Plasmid, MIDGE und MIDGE mit NLS gekoppelt (MIDGE-NLS). Dabei zeigt sich MIDGE-NLS als mit großem Abstand effektivstes Expressionssystem.

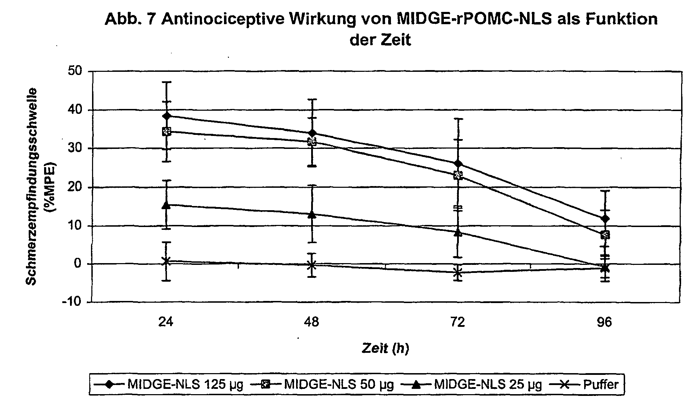

**Abb. 7** zeigt die antinociceptive Wirkung der peptidmodifizierten MIDGES in Abhängigkeit von der Zeit. Ein signifikanter Effekt der Schmerzlinderung ist selbst nach 96 Stunden bei applizierten Konzentrationen von MIDGE-rPOMC-NLS von 50 und 125 µg zu sehen.

### Ausführungsbeispiele

### Beispiel 1.1: Klonierung von rPOMC der Ratte

RNA wurde aus Rattenhirn-Zellen isoliert und mit Hilfe der reversen Transkriptase mit universellen Primem in DNA umgeschrieben. Mit spezifischen Primern für die cDNA des POMC (linker Primer: 5'-AATTATGGTACCATGCCGAGATTCTGCTACAG; rechter Primer: 5'-TTCTCAGAGCTCTCACTGGCCCTTCTTGTGCACGTTCTTGATG) wurde eine PCR durchgeführt. Das entstandene PCR-Produkt erwarteter Länge wurde aufgereinigt, mit den Restriktionsenzymen Kpnl und Sacl verdaut und in den Vektor pMOK kloniert. Klone wurden mit Restriktionsverdauen analysiert und Klone richtiger Fragmentlängen durch Sequenzierung bestätigt. Die Wildtyp-Sequenz ist in Seq.ID 3 (rPOMC-WT) wiedergegeben.

### Beispiel 1.2: Klonierung von rPOMC-β-END

Als Templat für die Klonierung des POMC-β-END diente das Plasmid pMOK-rPOMC. Durch PCR wurde ein Fragment amplifiziert, das im Vergleich zur POMC Sequenz um die kodierende Sequenz für das β-END verkürzt war. Das Stop-Codon für die neu entstandene verkürzte Sequenz wurde durch die PCR neu eingeführt (das Stop-Codon ist im rechten Primer fett markiert). Folgende Primer wurden in der PCR verwendet:

Nach Aufreinigen des PCR-Produktes und Restriktionsverdau mit Kpnl und Sacl wurde das Fragment in den Vektor pMOK kloniert. Klone wurden mit Restriktionsverdauen analysiert und Klone richtiger Fragmentlängen durch Sequenzierung bestätigt. Die Sequenz ist in Seq.ID 4 (rPOMC-β-END) wiedergegeben.

### Beispiel 1.3: Klonierung von rPOMC 1xβ-END

Als Template für die Klonierung des POMC 1xβEND diente das Plasmid pMOK-rPOMC. Das künstliche Genkonstrukt setzt sich zusammen aus den Nukleotiden der POMC-Sequenz bis zum letzten Codon vor dem Start der ACTH-Sequenz und der β-END-Sequenz. Zum Zusammensetzen der Gensequenz waren zwei PCR-Reaktionen nötig. Folgende Primer wurden verwendet:
Fragment 1:
Fragment 2:
Nach Amplifikation des ersten Fragmentes mit PCR und anschließender Aufreinigung wurde das Fragment mit Kpnl und Sacl geschnitten und in den Vektor pMOK kloniert. Richtige Klone wurden mit Restriktionsverdauen gefunden und mit Sequenzierung bestätigt. Das resultierende Plasmid wurde mit Bbsl und SacI geschnitten. Fragment 2 wurde nach Aufreinigung mit Eco31I und Sacl geschnitten. Die Überhänge, die von Bbsl und Eco31I generiert wurden, sind komplementär zueinander. Das Fragment 2 wurde in den Vektor mit Fragment 1 kloniert und richtige Klone mit Restriktionsverdauen gefunden und mit Sequenzierung bestätigt. Die Sequenz ist in Seq.ID 1 (rPOMC 1xβ-END) wiedergegeben.

### Beispiel 1.4: Klonierung von rPOMC 3xβ-END

Als Template für die Klonierung des POMC 3xβ-END diente ebenfalls das rPOMC. Im Vergleich zu Beispiel 1.3 musste eine weiteres Fragment durch Amplifikation mit PCR hergestellt werden (Fragment 3). Im Unterschied zu Fragment 2 aus Beispiel 1.3 enthielt die Sequenz des Fragmentes 3 kein Stopcodon am Ende der β-END-Sequenz. Das Zwischenprodukt und das Fragment 2 aus Beispiel 1.3 konnten hier verwendet werden. Folgende Primer wurden für die Amplifikation von Fragment 3 verwendet:
Fragment 3:
Nach Aufreinigung wurde das Fragment 3 mit Eco31I und Sacl geschnitten und in das mit Bbsl und Sacl geschnittene Zwischenprodukt kloniert. Das entstandene Zwischenprodukt 2 wurde ebenfalls mit Bbsl und SacI geschnitten und ein weiteres

Nach Aufreinigung wurde das Fragment 3 mit Eco31I und Sacl geschnitten und in das mit BbsI und SacI geschnittene Zwischenprodukt kloniert. Das entstandene Zwischenprodukt 2 wurde ebenfalls mit Bbsl und Sacl geschnitten und ein weiteres Mal wurde das Fragment 3 in das Zwischenprodukt 2 kloniert. Zwischenprodukt 3 wurde noch einmal mit Bbsl und SacI geschnitten und das Fragment 2 aus Beispiel 1.3 wurde als letztes Fragment (dieses Fragment enthält das Stop-Codon) in das Zwischenprodukt 3 kloniert. Das entstandene Plasmid enthielt 3 β-END Sequenzen hintereinander. Die Sequenz ist in Seq.ID 2 (rPOMC 3xβ-END) wiedergegeben.

### Beispiel 1.5: Klonierung von CRF

RNA aus Rattenhim-Zellen wurde isoliert und mit Hilfe der reversen Transkriptase mit universellen Primem wurde die RNA in DNA umgeschrieben. Mit spezifischen Primem für die cDNA des Corticotropin-releasing-factor (CRF); linker Primer. 5'-TTAATAGGTACCATGCGGCTGCGGCTGCTG; rechter Primer: 5'-ATTATGAGCTCTCATTTCCCGATAATCTCCATC) wurde eine PCR durchgeführt. Das entstandene PCR-Produkt erwarteter Länge wurde aufgereinigt, mit den Restriktionsenzymen Kpnl und Sacl verdaut und in den Vektor pMOK kloniert. Klone wurden mit Restriktionsverdauen analysiert und Klone richtiger Fragmentlängen durch Sequenzierung bestätigt. Die Sequenz ist in Seq.ID 6 (CRF) wiedergegeben.

### Beispiel 1.6: Herstellung von MIDGE-rPOMC mit und ohne NLS

MIDGES sind lineare, kovalent geschlossene Expressionskassetten, die lediglich aus dem CMV Promotor, einem Intron, der entsprechenden Gensequenz und einer Polyadenylierungssequenz bestehen (vgl. EP 0 941 318 B1). Die Konstrukte wurden wie folgt erhalten: das unter Beispiel 1.1 beschriebene Plasmid pMOK-rPOMC wurde mit Eco 31I vollständig verdaut. Die Ligation mit 5' phosphorylierten haamadelförmigen Oligodesoxynuklentiden (ODN) 5'-AGGGGTCCAG-TTTTCTGGAC-3' durch T4 DNA Ligase in Anwesenheit von Eco 31I wurde durch Erhitzen auf 70 °C gestoppt. Das resultierende Gemisch wurde konzentriert und mit Eco 31I und T7 DNA Ligase in Abwesenheit von Deoxyribonukleotid-Triphosphaten behandelt. Die Aufreinigung erfolgte durch Anionenaustauschchromatographie. MIDGES mit NLS Kopplung wurden wie folgt konstruiert: das NLS Peptid PKKKRKVEDPYC wurde in zwei Schritten an die ODN's gekoppelt. Zuerst wurde das modifizierte Oligonukleotid 5'-PH-d(GGGAGTCCAGT xT TTCTGGAC, wobei xT steht für amminomodifizierte Thyminbase mit C₂- Amminolinker) (0,1mM) mit sulfo-KMUS (5mM) in PBS bei Raumtemperatur (RT) aktiviert. Nach 120 min. wurde die Reaktion mit 50 mM Tris-(Hydroxymethyl)-Aminomethane gestoppt und das aktivierte ODN nach Ethanolpräzipation (300mM NaOAc pH 5.2, 5.5 mM MgCl₂, 70 % Ethanol), Zentrifugation und einmaligem Waschen mit 70% Ethanol erhalten. Das so erhaltene ODN (0,1mM) wurde in PBS gelöst und reagierte mit dem Peptid (0,2mM) für eine Stunde bei Raumtemperatur. Die Reaktion wurde durch Gelektrophorese (3%) und Ethidiumbromidfärbung überprüft. Das entstandene NLS gekoppelte ODN wurde durch HPLC aufgereinigt und zur Synthese der MIDGE-POMC-NLS Konstrukte wie zuvor beschrieben verwendet.

### Beispiel 2.1: Schmerzdämpfung nach Injektion von Expressionskonstrukten für rPOMC

50, 100, 250 und 350 µg pMOK-rPOMC wurden in 150mM Natriumphosphat pH 7,2 in einem Volumen von 200 µl in eine entzündete Rattenpfote injiziert. 24 h später wurde die Schmerzdämpfung in der entzündeten Rattenpfote untersucht. Die dabei verwendete Methode des "paw pressure threshold" ist u.a. in Schaefer et al., Proc. Nat. Acad. Sci USA 914219-4223 (1994) auf Seite 4220 im Methodenteil beschrieben. Abb. 3 zeigt die Ergebnisse des Versuches. Pro Gruppe wurden 6 Ratten verwendet.

## Patentansprüche

1. Arzneimittel zur Behandlung, insbesondere Verminderung oder Unterdrückung, der Schmerzempfindung bei höheren Tieren, insbesondere Menschen, enthaltend - unter Ausschluss von Zellen oder Zelllysaten - Expressionskonstrukte der POMC-Sequenz, aus welcher die kodierenden Abschnitte für die Gewebshormone adrenocorticotropes Hormon (ACTH) und/oder beta-melanozytenstimulierendes Hormon (beta-MSH) deletiert wurden.

2. Arzneimittel nach Anspruch 1, welches Expressionskonstrukte enthält, die einfach, zweifach und/oder dreifach für β-Endorphin kodieren.

3. Arzneimittel nach Anspruch 1 oder 2, welches zusätzlich noch Expressionskonstrukte für den Corticotropin-Releasing-Factor (CRF) enthält.

4. Arzneimittel nach einem der Ansprüche 1 bis 3, wobei es sich bei den DNA-Expressionskonstrukten um Plasmid-DNA oder um ein linear-kovalent geschlossenes DNA-Expressionskonstrukte handelt.

5. Arzneimittel nach Anspruch 4, wobei ein linear-kovalent geschlossenes DNA-Expressionskonstrukt mit einem Peptid konjugiert ist.

6. Arzneimittel nach Anspruch 5, wobei das linear-kovalent geschlossene DNA-Expressionskonstrukt mit einem die Kernlokalisationssequenz (NLS) des large T-Antigens von SV40 enthaltenden Peptid modifiziert ist.

7. Arzneimittel nach Anspruch 6, wobei das NLS-Peptid die Aminosäuresequenz PKKKRKVEDPYC aufweist.

8. Arzneimittel nach Anspruch 4 oder 5, wobei das linear-kovalent geschlossene DNA-Expressionskonstrukt mit einem kationischen Peptid bestehend aus 8 bis 20 Aminosäuren konjugiert ist.

9. Arzneimittel nach einem der Ansprüche 4 bis 8, wobei die Expressionskonstrukte durch Polyethylenimin komplexiert sind.

10. Arzneimittel nach einem der vorhergehenden Ansprüche, welches in einer injektionsfähigen Applikationsform vorliegt.

11. Vektor zur Herstellung eines DNA-Expressionskonstruktes als Bestandteil eines Arzneimittels nach den Ansprüchen 1 bis 10, enthaltend die POMC-Sequenz, welcher die kodierenden Abschnitte für die Gewebshormone adrenocorticotropes Hormon (ACTH) und/oder beta-melanozytenstimulierendes Hormon (beta-MSH) deletiert wurden, welches jedoch einmal den für β-Endorphin (β-END) kodierenden Sequenzabschnitt (rPOMC 1xβ-END: Seq.ID 1) enthält.

12. Vektor zur Herstellung eines DNA-Expressionskonstruktes als Bestandteil eines Arzneimittels nach den Ansprüchen 1 bis 10, enthaltend die POMC-Sequenz, welcher die kodierenden Abschnitte für die Gewebshormone adrenocorticotropes Hormon (ACTH) und/oder beta-melanozytenstimulierendes Hormon (beta-MSH) deletiert wurden, welches zweimal den für β-Endorphin (β-END) kodierenden Sequenzabschnitt (rPOMC 2xβ-END: Seq.ID 7) enthält.

13. Vektor zur Herstellung eines DNA-Expressionskonstruktes als Bestandteil eines Arzneimittels nach den Ansprüchen 1 bis 10, enthaltend die POMC-Sequenz, welcher die kodierenden Abschnitte für die Gewebshormone adrenocorticotropes Hormon (ACTH) und/oder beta-melanozytenstimulierendes Hormon (beta-MSH) deletiert wurden, welches dreimal den für β-Endorphin (β-END) kodierenden Sequenzabschnitt (rPOMC 3xβ-END: Seq.ID 2) enthält.

14. Vektor zur Herstellung eines DNA-Expressionskonstruktes als Bestandteil eines Arzneimittels nach den Ansprüchen 1 bis 10, enthaltend die Desoxyribonukleinsäuresequenz kodierend für Corticotropin-releasing-factor (CRF: Seq.ID 6).

15. Desoxyribonukleinsäuresequenz, enthaltend eine der für β-Endorphin kodierenden Sequenzabschnitte des Pro-Opiomelanocortingens (POMC), nämlich die in Seq.ID 1 (rPOMC 1xβ-END) wiedergegebene Sequenz.

16. Desoxyribonukleinsäuresequenz, enthaltend zwei der für β-Endorphin kodierenden Sequenzabschnitte des Pro-Opiomelanocortingens (POMC), nämlich die in Seq.ID 7 (rPOMC 2xβ-END) wiedergegebene Sequenz.

17. Desoxyribonukleinsäuresequenz, enthaltend drei der für β-Endorphin kodierenden Sequenzabschnitte des Pro-Opiomelanocortingens (POMC), nämlich die in Seq.ID 2 (rPOMC 3xβ-END) wiedergegebene Sequenz.

## Claims

1. Remedy for the reduction or suppression of the sensation of pain in higher animals, especially human beings, containing - with the exclusion of cells or cell lysates - expression constructs containing the POMC-sequence deleted of the coding regions for adrenocorticotropic hormone (ACTH) and/or beta-melanocyte stimulating hormone (β-MSH).

2. Remedy according to claim 1, containing expression constructs, which encode once, twice or three times for β-Endorphin.

3. Remedy according to claim 1 or 2, which contains additionally expression constructs for corticotropine-releasing-factor (CRF).

4. Remedy according to one of the claims 1 to 3, where the expression construct is a plasmid or a linear, covalently closed expression construct.

5. Remedy according to claim 4, where a linear, covalently closed expression construct is conjugated with a peptide.

6. Remedy according to claim 5, where the linear, covalently closed expression construct is modified with a peptide comprising the nuclear localization sequence (NLS) of the large T-antigen of SV40.

7. Remedy according to claim 6, where the NLS peptide contains the amino acid sequence PKKKRKVEDPYC.

8. Remedy according to claim 4 or 5, where the linear, covalently closed expression construct is conjugated to a cationic peptide of between 8 and 20 amino acids in length.

9. Remedy according to one of the claims 4 to 8, where the DNA is complexed by polyethylenimine.

10. Remedy according to one of the previous claims, which is applicable by injection.

11. Vector for the production of a DNA expression construct as a component of a remedy according to the claims 1 to 10, containing the POMC-sequence deleted of the coding regions for the tissue specific hormones adrenocorticotropic hormone (ACTH) and/or beta-melanocyte stimulating hormone (β-MSH), which contains at least once the coding sequence section (rPOMC 1xβ-END: Seq.ID1) for β-endorphin (β-END).

12. Vector for the production of an expression construct as a component of a remedy according to the claims 1 to 10, containing the POMC-sequence deleted of the coding regions for the tissue specific hormones adrenocorticotropic hormone (ACTH) and/or beta-melanocyte stimulating hormone (β-MSH), which contains at least twice the coding sequence section (rPOMC 1xβ-END: Seq.ID1) for β-endorphin (β-END).

13. Vector for the production of an expression construct as a component of a remedy according to the claims 1 to 10, containing the POMC-sequence deleted of the coding regions for the tissue specific hormones adrenocorticotropic hormone (ACTH) and/or beta-melanocyte stimulating hormone (β-MSH), which contains at least three times the coding sequence section (rPOMC 1xβ-END: Seq.ID1) for β-endorphin (β-END).

14. Vector for the production of an expression construct as a component of a remedy according to the claims 1 to 10, containing the desoxynucleic acid sequence coding for corticotropin releasing factor (CRF Seq.ID 6).

15. Desoxyribonucleic acid sequence, containing one of the sequence sections encoding β-endorphin from the pro-opiomelanocotrin gene (POMC), specifically the sequence shown in Seq.ID 1 (rPOMC 1xβ-END).

16. Desoxyribonucleic acid sequence, containing two of the sequence sections encoding β-endorphin from the pro-opiomelanocotrin gene (POMC), specifically the sequence shown in Seq.ID 7 (rPOMC p2xβ-END).

17. Desoxyribonucleic acid sequence, containing three of the sequence sections encoding β-endorphin from the pro-opiomelanocotrin gene (POMC), specifically the sequence shown in Seq.ID 2 (rPOMC 3xβ-END).

## Revendications

1. Médicament destiné à traiter, en particulier à réduire ou réprimer, la sensation de douleur chez les animaux supérieurs, en particulier les hommes, et contenant - à l'exclusion de cellules ou de lysats cellulaires - des constructs d'expression de la séquence de POMC de laquelle les segments codant pour les hormones tissulaires à savoir, l'hormone adrénocorticotrope (ACTH) et/ou l'hormone mélanocytostimulante (bêta-MSH) ont été supprimés.

2. Médicament selon la revendication 1, qui contient des constructs d'expression qui codent une fois, deux fois et/ou trois fois pour la β-endorphine.

3. Médicament selon la revendication 1 ou 2, qui contient en outre encore des constructs d'expression pour le facteur de libération de la corticotropine (CRF).

4. Médicament selon l'une des revendications 1 à 3, dans lequel les constructs d'expression d'ADN sont un ADN de plasmide ou un construct d'expression d'ADN fermé de façon linéaire-covalente.

5. Médicament selon la revendication 4, dans lequel un construct d'expression d'ADN fermé de façon linéaire-covalente est conjugué à un peptide.

6. Médicament selon la revendications 5, dans lequel le construct d'expression d'ADN fermé de façon linéaire-covalente est modifié avec un peptide contenant la séquence NLS (nucleus localisation signal) de l'antigène large T de SV40.

7. Médicament selon la revendication 6, dans lequel le peptide NLS comporte la séquence d'acides aminés PKKKRKVEDPYC.

8. Médicament selon la revendication 4 ou 5, dans lequel le construct d'expression d'ADN fermé de façon linéaire-covalente est conjugué à un peptide cationique composé de 8 à 20 acides aminés.

9. Médicament selon l'une des revendications 4 à 8, dans lequel les constructs d'expression sont complexés par une polyéthylèneimine.

10. Médicament selon l'une des revendications précédentes qui se présente sous une forme d'application injectable.

11. Vecteur pour la fabrication d'un construct d'expression d'ADN en tant que composant d'un médicament selon les revendications 1 à 10, contenant la séquence de POMC de laquelle les segments codant pour les hormones tissulaires à savoir, l'hormone adrénocorticotrope (ACTH) et/ou l'hormone bêta mélanocytostimulante bêta-MSH ont été supprimés, et qui contient cependant une fois le segment de séquence (rPOMC 1xβ-END : ID seq. 1) codant pour la β-endorphine (β-END).

12. Vecteur pour la fabrication d'un construct d'expression d'ADN en tant que composant d'un médicament selon les revendications 1 à 10, contenant la séquence de POMC de laquelle les segments codant pour les hormones tissulaires à savoir, l'hormone adrénocorticotrope, (ACTH) et/ou l'hormone bêta mélanocytostrimulante (bêta-MSH) ont été supprimés, et qui contient deux fois le segment de séquence (rPOMC 2xβ-END : ID seq. 7) codant pour la β-endorphine (β-END).

13. Vecteur pour la fabrication d'un construct d'expression d'ADN en tant que composant d'un médicament selon les revendications 1 à 10, contenant la séquence de POMC de laquelle les segments codant pour les hormones tissulaires à savoir l'hormone adrénocorticotrope, (ACTH) et/ou l'hormone bêta mélanocytostimulante (bêta-MSH) ont été supprimés, et qui contient encore trois fois le segment de séquence (rPOMC 3xβ-END : ID seq. 2) codant pour la β-endorphine (β-END).

14. Vecteur pour fabrication d'un construct d'expression d'ADN en tant que composant d'un médicament selon les revendications 1 à 10, contenant la séquence d'acide désoxyribonucléique codant pour le facteur de libération de la corticotropine (CRF : ID seq. 6).

15. Séquence d'acide désoxyribonucléique contenant un des segments de séquence du gène de pro-opiomélanocortine (POMC) codant pour la β-endorphine, à savoir la séquence reflétée dans l'ID seq. 1 (rPOMC 1xβ-END).

16. Séquence d'acide désoxyribonucléique contenant deux des segments de séquence du gène de pro-opiomélanocortine (POMC) codant pour la β-endorphine, à savoir la séquence reflétée dans l'ID seq. 7 (rPOMC 2xβ-END).

17. Séquence d'acide désoxyribonucléique contenant trois des segments de séquence du gène de pro-opiomélanocortine (POMC) codant pour la β-endorphine, à savoir la séquence reflétée dans l'ID seq. 2 (rPOMC 3xβ-END).
